# EUROPEAN PATENT SPECIFICATION

(11) **EP 2 245 454 B1**
(45) Date of publication and mention of the grant of the patent: **24.06.2015**
(21) Application number: 09714556.9
(22) Date of filing: 25.02.2009
(51) Int. Cl.: G01N 33/487

(54) **MICRO-ELECTRODE ARRAY BASED ON OPTICALLY TRANSPARENT POLYMERIC CONDUCTIVE MATERIALS, AND METHOD FOR THE MANUFACTURING THEREOF**
MIKROELEKTRODEN-ARRAY AUF BASIS OPTISCH TRANSPARENTER LEITFÄHIGER POLYMERMATERIALIEN UND VERFAHREN ZUR HERSTELLUNG DAVON
MATRICE DE MICROÉLECTRODES À BASE DE MATÉRIAUX CONDUCTEURS OPTIQUEMENT TRANSPARENTS, ET SON PROCÉDÉ DE FABRICATION

(30) Priority: 29.02.2008 IT TO20080152
(43) Date of publication of application: 03.11.2010
(73) Proprietor: Fondazione Istituto Italiano Di Tecnologia, 16163 Genova (IT)
(72) Inventor: BLAU, Axel, I-16145 Genova (IT); BENFENATI, Fabio, I-16167 Genova (IT)
(74) Representative: Deambrogi, Edgardo
(86) International application number: PCT/IB2009/050751
(87) International publication number: WO 2009/107069

(56) References cited:
- CLAVEROL-TINTURÉ E ET AL: "COMMUNICATION; Multielectrode arrays with elastomeric microstructured overlays for extracellular recordings from patterned neurons; Communication" JOURNAL OF NEURAL ENGINEERING, INSTITUTE OF PHYSICS PUBLISHING, BRISTOL, GB, vol. 2, no. 2, 1 June 2005 (2005-06-01), pages L1-L7, XP020093427 ISSN: 1741-2552
- HAFIZOVIC S ET AL: "A CMOS-based microelectrode array for interaction with neuronal cultures" JOURNAL OF NEUROSCIENCE METHODS, ELSEVIER SCIENCE PUBLISHER B.V., AMSTERDAM, NL, vol. 164, no. 1, 15 August 2007 (2007-08-15), pages 93-106, XP026021007 ISSN: 0165-0270 [retrieved on 2007-08-15]
- HEER F ET AL: "CMOS microelectrode array for bidirectional interaction with neuronal networks" SOLID-STATE CIRCUITS CONFERENCE, 2005. ESSCIRC 2005. PROCEEDINGS OF THE 31ST EUROPEAN, IEEE, PISCATAWAY, NJ, USA, 12 September 2005 (2005-09-12), pages 335-338, XP010854970 ISBN: 978-0-7803-9205-2
- FREY U ET AL: "Cell Recordings with a CMOS High-density Microelectrode Array" ENGINEERING IN MEDICINE AND BIOLOGY SOCIETY, 2007. EMBS 2007. 29TH ANNUAL INTERNATIONAL CONFERENCE OF THE IEEE, IEEE, PISCATAWAY, NJ, USA, 22 August 2007 (2007-08-22), pages 167-170, XP031336128 ISBN: 978-1-4244-0787-3

## Description

The present invention relates to a micro-electrode array, and to the corresponding manufacturing technique.

It is known that some cells, such as neurons or cardiac muscle cells (cardiomyocytes) communicate through biochemical signals and electric signals. In the case of the latter, they are signals the voltage of which ranges between some microvolts and some millivolts. Considering these electric signals as being equivalent to voltage or current sources, they can be detected through analysis techniques that are standard in the electric or electronic field.

However, since biologic cells are small, with diameters ranging between 10 and 100 micrometers, the arrangement of a sufficiently small voltage or current probe in the proximity of the cell the electric signals of which are to be analyzed is a problematic aspect.

Therefore, several electrophysiology technologies for the sampling of the bioelectric signals have been developed, comprising the use of needle electrodes, patch clamping, or the use of micro-electrode arrays.

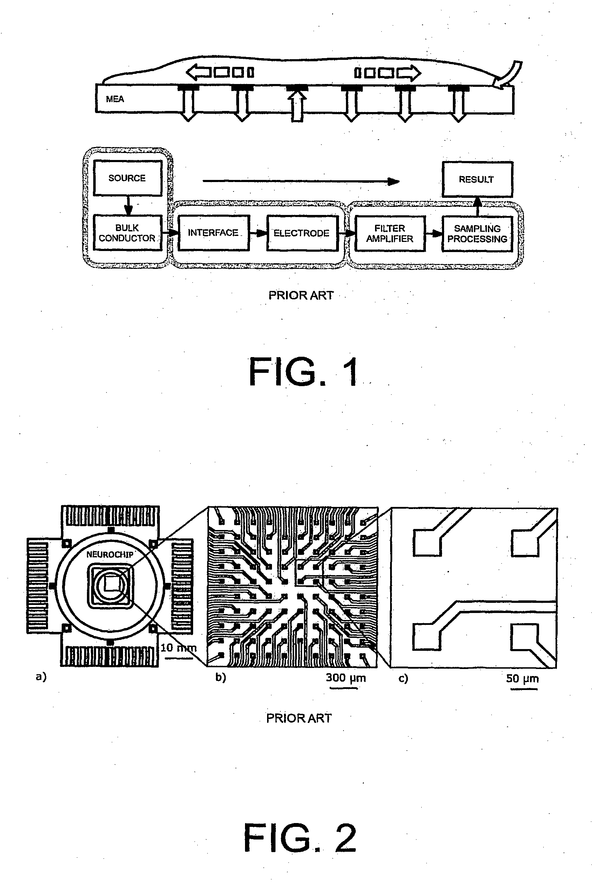

A schematic representation of the acquisition and/or stimulation of bioelectric signals through a micro-electrode array is shown in Fig. 1.

The micro-electrode arrays can essentially act according to two "dual" modes, detecting bioelectric signals emitted by a biological entity, or delivering preset electric pulses to influence biologic functions, respectively. They are considered "non-invasive" devices, since they do not penetrate the cells, thus damaging them, but they simply act by virtue of a contact.

In essence, the bioelectric signals (whether they are, for example, action or field potentials from neurons, brain slices, cardiomyocytes, or pancreatic beta cells), are detected in the form of capacitive currents which are drawn at microscopic contact terminals located in an interaction area with the biological entity to be analyzed, and are conducted through interconnection tracks to peripheral macroscopic contact pads, adapted to provide an electric connection with processing, amplification and filtering electronics.

In the dual operation mode, the preset stimulation voltage or current pulses for the entity under examination can be provided by external generating devices via the contact pads, thereby arriving to the micro-electrodes by covering an inverse path along the interconnection tracks, to stimulate the biological entities in contact therewith.

Micro-electrode arrays can be employed also for *in vivo* applications, for example, for the stimulation of organs (deep encephalon, retinal implants), artificial apparatus (cardiac pacemakers), or for the interconnection with the peripheral nervous systems for the control of prostheses.

According to the prior art, recording of the electrophysiological signals can be carried out by two different types of micro-electrode arrays: active arrays or passive arrays.

Examples of multielectrode arrays with elastomeric featured wells on glass substrates with patterned electrodes made from standard lithographic techniques are given in Journal of Neural Engineering 2.(2005) L1-L7, Claverol-Tinturé et al.

The micro-electrode active arrays comprise an electronics for amplification, filtering and/or pre-processing of signals which is integrated with the array. Traditionally, these arrays are manufactured in the CMOS or FET technology. The main advantages are the arrangement of a high number of micro-electrodes, the integration of the pre-processing electronics, which does not require the use of expensive external equipments, and the possibility of mass manufacturing. Unadvantageously, in order to admit the supplementary processing electronics according to the known technologies and integrated circuit architectures, such arrays are made of non-transparent materials for electronics applications, therefore the biological entities on which the arrays are located can be concomitantly studied only by means of epi-illumination or vertical incident light illumination microscopy.

The micro-electrode passive arrays comprise micro-electrodes circuit arrangements used only for the transduction of the bioelectric signals, that is, without further pre-processing. Such arrays are adapted to conduct the bioelectric signals via the interconnection tracks to the outer contact pads, where they are subsequently amplified and analyzed by external electronics.

Advantageously, passive arrays have a quite simple layout. They consist in a support material on which metal electrode terminals, interconnection tracks, and micromachined contact pads are defined via photolithographic processes, as shown according to an illustration in different scales in Fig. 2. Typical support materials are glass or polymers such as, for example, polyimide. In order to deposit and pattern the metallic tracks, vapour phase deposition or screen printing techniques are employed. Gold, platinum, palladium, iridium, or tin and indium oxide are the metal materials typically employed as conductors for manufacturing the electrodes. The diameters of the micro-electrodes range between 10 and 100 microns, and the spacing therebetween typically ranges between 10 and 500 microns.

In some cases, the electrodes are modified by means of a sputtering deposition of titanium or iridium oxide emitters, or by fractal platinum black electrodeposition to enlarge the effective surface area thereof and/or the charge delivery capacity thereof.

The interconnection conductive tracks which connect the micro-electrodes to the macroscopic contact pads to convey the electric signal from its source to a position in which it is processed or used, are insulated so as to avoid losses of signal in the path between the micro-electrodes and the contact pads. Typical insulating materials are glass (SiO₂), silicon nitride (Si₃N₄), or non-conductive polymers, such as polyimide.

With the aim of being able to use microscopy techniques in order to study the biological entities subjected to the electrophysiological analysis, it is desirable that the materials constituting the micro-electrode arrays have transparency and biocompatibility properties, typically having to contact a cell culture.

Therefore, object of the invention is to manufacture a passive micro-electrode array with transparency and biocompatibility characteristics, at low cost, by providing a relative mass production process which is rapid and also inexpensive, complying with the following constraints:
- the materials in contact with the cells or the cell culture environment have to be biocompatible, in order not to affect the cellular metabolism;
- the materials have to be biostable, in order to avoid modifications of the respective chemical, mechanical, physical, and electric properties, when in contact with the physiological environment of the application (for example, salts in nutrition solutions, cells metabolites, etc.);
- the materials have to allow the analysis of the biological entities through light microscopy;
- the array has to be inexpensive and easy to manufacture, since it is traditionally used in disposable applications, or anyway discarded after a reduced number of uses;
- the array does not have to contain hazardous wastes-generating materials.

Such objects are achieved through a micro-electrode array having the characteristics defined by claim 1.

It is a further object of the invention a method for the manufacturing of a micro-electrode array having the characteristics defined by claim 7.

Particular embodiments are the subject of the dependant claims, and they are considered to be an integrating part of the present description.

In brief, the present invention is based on the principle of manufacturing a micro-electrode array starting from electrically conductive polymeric materials and electrically insulating polymeric materials which have properties of biocompatibility, biostability, and transparency to light radiation. Such materials can be processed by employing a two-layer master mould carrying predetermined microstructures for the fabrication of a substrate of the array, preferably via a replica molding technology.

The micro-electrode array substrate is made of insulating material, preferably transparent polydimethylsiloxane (PDMS). The conductive materials for the electrodes, the interconnection tracks, and the contact pads are transparent polymeric materials, preferably organic polymeric materials, such as poly(3,4-ethylenedioxythiophene) (PEDOT).

It is shown that all the materials are biocompatible, biostable, and optically transparent.

Advantageously, by virtue of the selection of such materials, the production process steps for the array can be automated.

By preferably adopting a replica molding technique, an insulating crosslinkable polymer, such as polydimethylsiloxane, is poured onto a previously patterned printing master, subjected to crosslinking, and peeled off. The support thus obtained has template cavities of the micro-electrode array, which are filled with a conductive polymeric liquid, such as poly(3,4-ethylenedioxythiophene), which fills in the channels and the apertures corresponding to the definition of the micro-electrodes by capillary diffusion. After drying the conductive polymer, the remaining open cavities are insulated by a mechanical support layer, for example, obtained by deposition of a second PDMS layer (in order to implement a wholly polymer-based micro-electrode array), or a glass film.

Therefore, such a micro-electrode array is an array of the passive type, since it does not provide for the implementation of signal processing electronic components or circuits. Such an array is adapted to transduce and direct biological signals emitted by a microscopic source, for example, a cell culture substrate in contact with the electrode areas of the array, to external macroscopic processing equipments, such as amplifiers, filters, and other data conversion equipments connected via the array connection pads.

Typically, the micro-electrodes thus manufactured have diameters ranging between 10 and 100 micrometers, and the connection pads have dimensions of the order of 1x2mm².

Further characteristics and advantages of the invention will be set forth in more detail in the following detailed description of an embodiment thereof, given by way of non-limitative example, with reference to the annexed drawings, in which:
Fig. 1 and Fig. 2 show a general circuit diagram for the acquisition and/or stimulation of bioelectric signals, and an illustration in different scales of a micro-electrode array, respectively, according to the prior art discussed in the introduction of the present description;
Fig. 3 shows a pair of photographic masks employed in manufacturing the micro-electrode array according to the method of the invention;
- Figs. 4a-4f are exemplary cross-sectional views of the different steps of the manufacturing process of a mould portion for the manufacturing of a micro-electrode array; and
- Figs. 5a-5e are exemplary cross-sectional views of the different steps of a manufacturing process of a micro-electrode array starting from the mould thus obtained.

In the following, a method of manufacturing a micro-electrode array by replica molding according to the invention is described in detail, the array including a substrate of transparent polymer, as well as a plurality of micro-electrodes, a plurality of respective interconnection tracks, and corresponding contact pads of transparent organic conductive polymer, and spaced one from the other by an insulating material based on a transparent polymer.

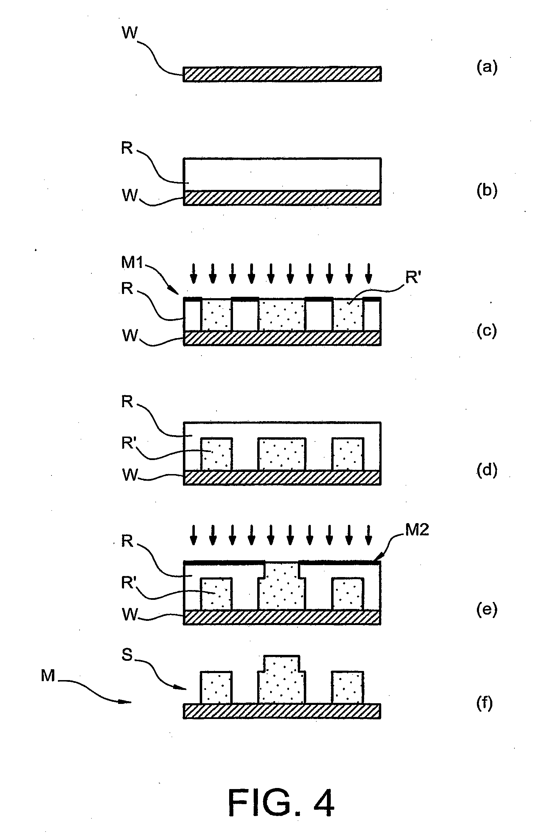

A master mould having in the negative the structures of the final micro-electrode array is obtained in a first step (Figs. 4a-4f). The master mould includes materials which have minimum abrasive properties in order to allow the repetitive moulding of a number of micro-electrode arrays.

Such a master can be produced, for example, by photolithographic patterning of photosensitive resin layers on a silicon wafer, but other production techniques can be employed, such as, for example, hot embossing or micro-milling techniques.

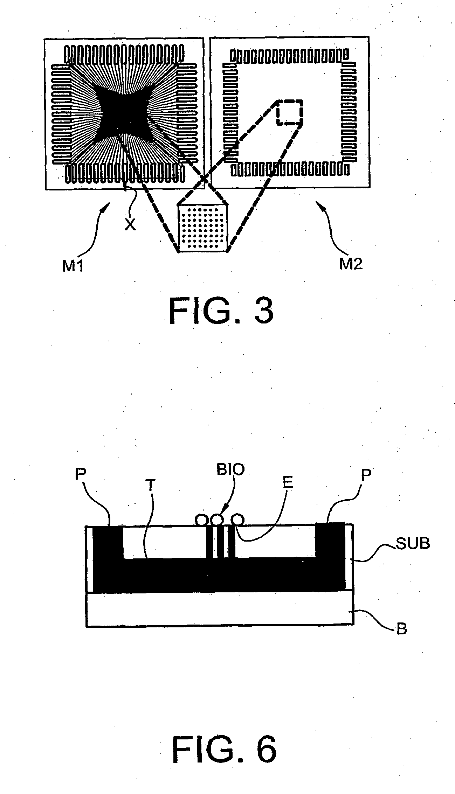

For the patterning thereof, two photo/repro-graphic masks M1 and M2 are employed as shown in Fig. 3. The first mask M1 reproduces the array central micro-electrodes, the inter-connection tracks which result buried in the end structure, and the outer contact pads, while the second mask M2 only reproduces the central micro-electrodes and the contact pads which have to result to be as exposed areas in the end structure.

As a function of the selection of the type of photosensitive resin (positive or negative), the image produced in the resin is the negative or positive image of the photomask (for example, in the case of a positive photosensitive resin, the resin illuminated areas are washed off, while the non-illuminated areas are maintained, that is, a positive resin produces a negative image of the mask).

The procedure detailed herein below describes the production of a master mould through photographic masks for negative photosensitive resins.

In this case, after the illumination of the resin through a predefined mask, the illuminated areas are subjected to crosslinking, thereby forming a configuration of relief microstructures which has a height which is equal to the resin layer initial thickness, while the non-illuminated areas are washed off in a successive washing or "development" step.

In the following, with reference to Figs. 4a-4f, the operative steps for generating a two-layer master by replica molding starting from a negative photographic resin on a silicon wafer are described.

A suitably cleaned silicon wafer W (Fig. 4a) is coated - by means, for example, of a spin-coating technique - with a first photosensitive resin layer R, for example, having a thickness ranging between 10 and 200 micrometers. Before being illuminated, the resin layer undergoes curing in a two-step heating process comprising, for example, a first heating step at 65 °C for a period of 10 minutes, followed by a second heating step at the temperature of 95 °C for a period of 30 minutes (Fig. 4b).

The first photomask M1 bearing the image of the three conductive components (electrodes, interconnection tracks, and contact pads) of the micro-electrode array is arranged in the cured state on the resin layer R. Then, the structure is subjected to illumination (Fig. 4c) with ultraviolet rays to reach a crosslinked condition.

By using a negative photosensitive resin, the illuminated areas stabilize by crosslinking (areas R').

The resin deposition and crosslinking process is repeated to grow the patterned resin layer (Figs. 4d, 4e), this time through the second photomask M2 bearing only the images of the electrodes and the outer contact pads.

It shall be apparent that the second mask has to be precisely aligned over the already produced first structure by means of suitable aligning references. In any case, the second resin layer thickness can be different from the first layer thickness.

In a successive annealing and development step, both resin layers are removed (Fig. 4f), revealing on the silicon wafer substrate the microstructures S defined by the transparent areas of the two photomasks through the thickness of the two respective resin layers, thus constituting the master mould M for the successive replica molding steps.

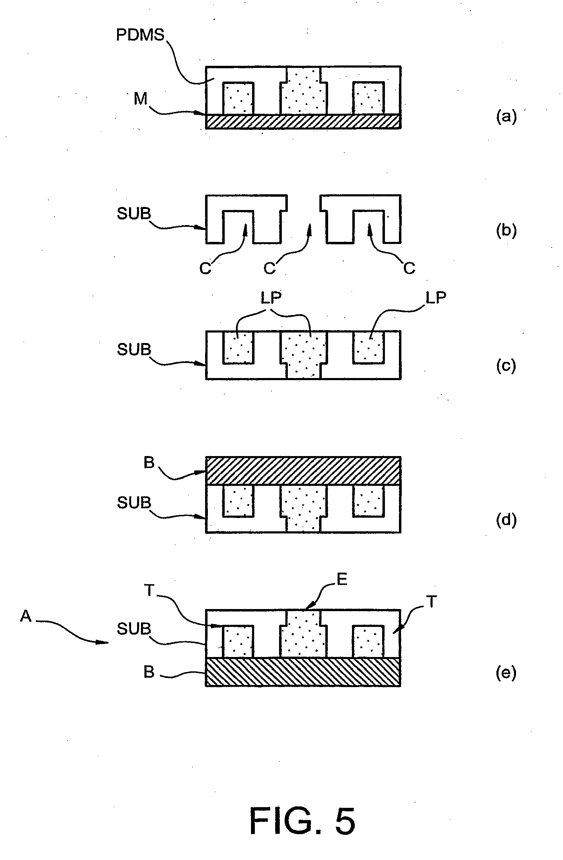

In Figs. 5a-5e, the manufacturing steps of a micro-electrode array A according to the preferred replica molding technique starting from the thus-manufactured master M, which can be employed for the printing of a virtually infinite number of polymeric micro-electrode arrays are shown in succession.

In Fig. 5a, a filling step of the mould M through a polydimethylsiloxane (PDMS) pre-polymeric viscous mixture, for example, the mixture known under the trade name Sylgard 184 manufactured by Dow Coming, is shown. Then, the mixture is subjected to crosslinking (for example, by heating), and the thus-obtained flexible polymer is peeled off from the master, thereby originating an armature of substrate SUB of the micro-electrode array (Fig. 5b) provided with cavities C at the location of the resin microstructures of the mould M.

Then, the above-mentioned cavities are filled (by overturning the substrate SUB armature) with an electrically conductive liquid polymeric solution LP, for example, the organic polymer poly(3,4-ethylenedioxythiophene) (PEDOT), known under the trade name Baytron P CPP 105D by Starck/Bayer, and dried by solvent evaporation (Fig. 5c). In this case, it shall be noted that the adhesion of the conductive polymer to the PDMS can be improved through a hydrophilization step of PDMS via an oxygen plasma.

Finally, the upper surface of the semi-finished structure, constituting the micro-electrode array rear side, is preferably coated (Fig. 5d) with an additional PDMS or glass film layer B, deposited by pipetting, screen printing, or ink jet printing, acting as a mechanical support layer for an enhanced stiffness of the array structure on the whole.

Finally, in Fig. 5e the completed structure of the micro-electrode array A, obtained by overturning the structure of Fig. 5d, is shown. The support layer B, the substrate SUB, the buried interconnection tracks T regions, and the surface electrodes E are shown in cross-section. Then the cells can be cultured on the upper side via standard cell culture protocols.

It shall be apparent that other manufacturing techniques are equally possible, for example, a hot embossing technique, whereby an insulating polymer-based substrate SUB, for example, polymethylmethacrylate (PMMA), is subjected to hot embossing by means of the mould M, without departing from the principles of the present invention.

In Fig. 6, a sectional, enlarged view of the thus-obtained array is shown, in which the conductive portions, emerging to the surface of the substrate SUB, of the micro-electrodes E and the contact pads P, and the buried conductive portions of the conductive tracks T are highlighted.

The conductors are electrically insulated from the outside, except for the signal source areas or signal withdrawal areas. These areas are arranged at opposite ends of each conductor, one of which being represented by a central micro-electrode E in the proximity of the biological entities BIO to be analyzed, the other one being a macroscopic contact pad P at the array outer edge.

It shall be apparent that, the principle of the invention being retained, the embodiments and the implementation details may be widely modified relative to what has been described and illustrated by way of non-limiting example only, without for this departing from the protection scope of the present invention, as defined by the annexed claims.

## Claims

1. A micro-electrode array (A), comprising an insulating substrate (SUB) and a plurality of conductive paths (E, T, P) defined therein, each of which includes:
- an electrode area (E) with micrometer dimensions, emerging to the array surface (A) in a first contact region with a biological sample to be analyzed or to stimulate;
- a connection area (P) with macroscopic dimensions, emerging to the array surface (A) in a second contact region for the electrical connection with external circuital arrangements for signal processing, adapted to generate stimulation electric signals to be conveyed to said electrode area (E) or to process electric signals collected by said electrode area (E) representative of the sample physiological activity; and
- a buried interconnection region (T) between said electrode area (E) and said connection area (P),
the array (A) being **characterized in that** said insulating substrate (SUB) is made of a first polymeric material, and said conductive paths (E, T, P) are made of a second polymeric material.

2. The micro-electrode array (A) according to claim 1, wherein said first and second polymeric materials are optically transparent.

3. The micro-electrode array according to claim 2, wherein said insulating polymeric material is polydimethylsiloxane (PDMS).

4. The micro-electrode array (A) according to claim 2, wherein said conductive polymeric material is poly(3,4-ethylenedioxythiophene) (PEDOT).

5. The micro-electrode array (A) according to any one of the preceding claims, comprising a mechanical support layer (B) for the substrate (SUB), arranged at a part opposite the emerging surface of the electrode areas (E) and the connection areas (P) of the conductive paths, wherein the support layer (B) is made of polydimethylsiloxane (PDMS).

6. The micro-electrode array (A) according to any one of the preceding claims, comprising a mechanical support layer (B) for the substrate (SUB), arranged at a part opposite the emerging surface of the electrode areas (E) and the connection areas (P) of the conductive paths, wherein the support layer (B) is made of glass.

7. A method for the manufacturing of a micro-electrode array (A), which comprises an insulating substrate (SUB) and a plurality of conductive paths (E, T, P) defined therein, each of which includes:
- an electrode area (E) with micrometer dimensions, emerging to the array surface (A) in a first contact region with a biological sample to be analyzed or to stimulate;
- a connection area (P) with macroscopic dimensions, emerging to the array surface (A) in a second contact region for the electrical connection with external circuital arrangements for signal processing, adapted to generate stimulation electric signals to be conveyed to said electrode area (E), or to process electric signals collected by said electrode area (E) representative of the sample physiological activity; and
- a buried interconnection region (T) between said electrode area (E) and said connection area (P),
the method comprising the steps of:
- configuring an insulating substrate (SUB) volume of a first polymeric material through a prearranged master mould (M) bearing a configuration of microstructures (S) adapted to define a plurality of electrode areas (E), corresponding connection areas (P) and relative interconnection regions (T) in the substrate (SUB) volume, in the form of cavities (C);
- filling said cavities (C) with a second conductive polymeric material so as to form said conductive paths (E, T, P); and
- arranging an insulating material sealing layer (B) for said filled cavities (C).

8. The method according to claim 7, wherein the configuration of an insulating substrate (SUB) volume takes place by replica molding, and includes the following steps:
- deposition of the first material in a pre-polymeric viscous form in the mould (M);
- crosslinking of the material volume;
- separation of the substrate (SUB) volume from the mould (M), so as to make cavities (C) formed therein by the configuration of relief microstructures (S) of the mould (M) accessible; and
- fill in of the cavities (C) with the second material in pre-polymeric liquid form by capillarity diffusion, and successive curing of the material.

9. The method according to claim 7, wherein the insulating polymeric material is polydimethylsiloxane (PDMS).

10. The method according to claim 9, comprising a hydrophilization step of PDMS by means of an oxygen plasma.

11. The method according to claim 7, wherein the conductive polymeric material is poly(3,4-ethylenedioxythiophene) (PEDOT).

12. The method according to claim 7, wherein the master mould (M) is obtained by photolithographic structuring of a first photosensitive resin layer (R) for the configuration of buried conductive areas (T) of the array (A) and, subsequently, of a second overlapped photosensitive resin layer (R) for the configuration of exposed conductive areas (AND, P) of the array (A).

13. The method according to claim 12, wherein the manufacturing of the master mould (M) comprises:
- arranging a support substrate (W);
- depositing a first photosensitive resin layer (R) on the substrate (W);
- illuminating the deposited resin layer (R) through a first photomask (M1) bearing the image of said electrode areas (E), corresponding connection areas (P) and relative interconnection regions (T), to reach a crosslinked condition (R');
- depositing a second photosensitive resin layer (R) on the first layer;
- illuminating the deposited resin layer (R) through a second photomask (M2) in an aligned condition, and bearing the image of said electrode areas (E) and corresponding connection areas (P), to reach a crosslinked condition (R'); and
- developing and selectively removing the non-crosslinked resin layers (R), so as to have said configuration of microstructures (S) in relief on the support substrate (W).

## Patentansprüche

1. Mikroelektroden-Array (A), umfassend ein Isoliersubstrat (SUB) und mehrere darin festgelegte Leiterbahnen (E, T, P), die jeweils einschließen:
- einen Elektrodenbereich (E) mit Mikrometerdimensionen, der an der Arrayoberfläche (A) in einer ersten Kontaktregion mit einer zu analysierenden oder zu stimulierenden biologischen Probe zutage tritt;
- einen Verbindungsbereich (P) mit makroskopischen Dimensionen, der an der Arrayoberfläche (A) in einer zweiten Kontaktregion für die elektrische Verbindung mit externen Schaltungsanordnungen für Signalverarbeitung zutage tritt, ausgelegt zum Erzeugen elektrischer Stimulationssignale, die dem Elektrodenbereich (E) zuzuführen sind, oder zum Verarbeiten durch den Elektrodenbereich (E) aufgefangener elektrischer Signale, die repräsentativ für die physiologische Probenaktivität sind;
- eine vergrabene Region (T) zur gegenseitigen Verbindung, zwischen dem Elektrodenbereich (E) und dem Verbindungsbereich (P),
wobei der Array (A) **dadurch gekennzeichnet ist, dass** das Isoliersubstrat (SUB) aus einem ersten polymeren Material gefertigt ist, und die Leiterbahnen (E, T, P) aus einem zweiten polymeren Material gefertigt sind.

2. Mikroelektroden-Array (A) nach Anspruch 1, wobei das erste und das zweite polymere Material optisch transparent sind.

3. Mikroelektroden-Array nach Anspruch 2, wobei das isolierende polymere Material Polydimethylsiloxan (PDMS) ist.

4. Mikroelektroden-Array (A) nach Anspruch 2, wobei das leitende polymere Material Poly(3,4-ethylendioxythiophen) (PEDOT) ist.

5. Mikroelektroden-Array (A) nach einem der vorstehenden Ansprüche, umfassend eine mechanische Trägerschicht (B) für das Substrat (SUB), angeordnet an einem Teil gegenüber der zutage tretenden Fläche der Elektrodenbereiche (E) und der Verbindungsbereiche (P) der Leiterbahnen, wobei die Trägerschicht (B) aus Polydimethylsiloxan (PDMS) gefertigt ist.

6. Mikroelektroden-Array (A) nach einem der vorstehenden Ansprüche, umfassend eine mechanische Trägerschicht (B) für das Substrat (SUB), angeordnet an einem Teil gegenüber der zutage tretenden Fläche der Elektrodenbereiche (E) und der Verbindungsbereiche (P) der Leiterbahnen, wobei die Trägerschicht (B) aus Glas gefertigt ist.

7. Verfahren für die Herstellung eines Mikroelektroden-Arrays (A), der ein Isoliersubstrat (SUB) und mehrere darin festgelegte Leiterbahnen (E, T, P) umfasst, die jeweils einschließen:
- einen Elektrodenbereich (E) mit Mikrometerdimensionen, der an der Arrayoberfläche (A) in einer ersten Kontaktregion mit einer zu analysierenden oder zu stimulierenden biologischen Probe zutage tritt;
- einen Verbindungsbereich (P) mit makroskopischen Dimensionen, der an der Arrayoberfläche (A) in einer zweiten Kontaktregion für die elektrische Verbindung mit externen Schaltungsanordnungen für Signalverarbeitung zutage tritt, ausgelegt zum Erzeugen elektrischer Stimulationssignale, die dem Elektrodenbereich (E) zuzuführen sind, oder zum Verarbeiten durch den Elektrodenbereich (E) aufgefangener elektrischer Signale, die repräsentativ für die physiologische Probenaktivität sind;
- eine vergrabene Region (T) zur gegenseitigen Verbindung, zwischen dem Elektrodenbereich (E) und dem Verbindungsbereich (P),
wobei das Verfahren die Schritte umfasst:
- Konfigurieren eines Isoliersubstrat- (SUB-) Volumens eines ersten polymeren Materials durch eine vorab angeordnete Mutterform (M), die eine Konfiguration von Mikrostrukturen (S) trägt, angepasst zur Festlegung mehrerer Elektrodenbereiche (E), entsprechender Verbindungsbereiche (P) und relativer Regionen (T) zur gegenseitigen Verbindung im Substrat- (SUB-) Volumen, in Form von Kavitäten (C);
- Füllen der Kavitäten (C) mit einem zweiten leitenden polymeren Material, um die Leiterbahnen (E, T, P) zu bilden; und
- Anordnen einer Isoliermaterial-Abdichtschicht (B) für die gefüllten Kavitäten (C).

8. Verfahren nach Anspruch 7, wobei die Konfiguration eines Isoliersubstrat- (SUB-) Volumens durch Replica-Molding stattfindet, und die folgenden Schritte einschließt:
- Deponierung des ersten Materials in einem präpolymeren viskosen Zustand in der Form (M);
- Vernetzung des Materialvolumens;
- Trennung des Substrat- (SUB-) Volumens von der Form (M), um Kavitäten (C) zugänglich zu machen, die darin durch die Konfiguration von Reliefmikrostrukturen (S) der Form (M) ausgebildet sind; und
- Ausfüllen der Kavitäten (C) mit dem zweiten Material in präpolymerem flüssigem Zustand durch Kapillardiffusion, und sukzessives Aushärten des Materials.

9. Verfahren nach Anspruch 7, wobei das isolierende polymere Material Polydimethylsiloxan (PDMS) ist.

10. Verfahren nach Anspruch 9, das einen PDMS-Hydrophilierungsschritt mittels eines Sauerstoffplasmas umfasst.

11. Verfahren nach Anspruch 7, wobei das leitende polymere Material Poly(3,4-Ethylendioxythiophen) (PEDOT) ist.

12. Verfahren nach Anspruch 7, wobei die Mutterform (M) erhalten wird durch photolithographische Strukturierung einer ersten photosensitiven Harzschicht (R) für die Konfiguration vergrabener leitender Bereiche (T) des Arrays (A) und anschließend einer zweiten überlappten photosensitiven Harzschicht (R) für die Konfiguration freiliegender leitender Bereiche (AND, P) des Arrays (A).

13. Verfahren nach Anspruch 12, wobei die Herstellung der Mutterform (M) umfasst:
- Anordnen eines Trägersubstrats (W);
- Aufbringen einer ersten photosensitiven Harzschicht (R) auf das Substrat (W);
- Beleuchten der aufgebrachten Harzschicht (R) durch eine erste Photomaske (M1), die das Bild der Elektrodenbereiche (E), entsprechender Verbindungsbereiche (P) und relativer Regionen (T) zur gegenseitigen Verbindung trägt, zwecks Erzielung eines vernetzten Zustands (R');
- Aufbringen einer zweiten photosensitiven Harzschicht (R) auf die erste Schicht;
- Beleuchten der aufgebrachten Harzschicht (R) durch eine zweite Photomaske (M2), die sich in ausgerichtetem Zustand befindet, und die das Bild der Elektrodenbereiche (E) und entsprechenden Verbindungsbereiche (P) trägt, zwecks Erzielung eines vernetzten Zustands (R'); und
- Entwickeln und selektives Entfernen der nicht vernetzten Harzschichten (R), um die Konfiguration von Mikrostrukturen (S) im Relief auf dem Trägersubstrat (W) zu erhalten.

## Revendications

1. Grille de micro-électrodes (A), comprenant un substrat isolant (SUB) et un ensemble de pistes conductrices (E, T, P) définies sur ce substrat, comprenant chacune :
- une zone d'électrode (E) de dimensions micrométriques, apparaissant en surface de la grille (A) dans une première zone de contact avec un échantillon biologique à analyser ou à stimuler,
- une zone de connexion (P) de dimensions macroscopiques, apparaissant en surface de la grille (A) dans une deuxième zone de contact pour le branchement électrique avec des circuits externes pour le traitement des signaux, adaptée pour produire des signaux électriques de stimulation à transporter jusqu'à ladite zone d'électrode (E) ou pour traiter des signaux électriques collectés par ladite zone d'électrode (E) représentatifs de l'activité physiologique de l'échantillon et
- une zone d'interconnexion incrustée (T) entre ladite zone d'électrode (E) et ladite zone de connexion (P),
la grille (A) étant **caractérisée en ce que** ledit substrat isolant (SUB) est constitué d'un premier polymère et lesdites pistes conductrices (E, T, P) sont constituées d'un deuxième polymère.

2. Grille de micro-électrodes (A) selon la revendication 1, dans laquelle lesdits premier et deuxième polymères sont optiquement transparents.

3. Grille de micro-électrodes selon la revendication 2, dans laquelle ledit polymère isolant est du polydiméthylsiloxane (PDMS).

4. Grille de micro-électrodes (A) selon la revendication 2, dans laquelle ledit polymère conducteur est du poly(3,4-éthylènedioxythiophène) (PEDOT).

5. Grille de micro-électrodes (A) selon une des revendications précédentes, comprenant une couche de support mécanique (B) pour le substrat (SUB), située à un endroit à l'opposé de la surface apparente des zones d'électrodes (E) et des zones de connexion (P) des pistes conductrices, dans laquelle la couche de base (B) est du polydiméthylsiloxane (PDMS).

6. Grille de micro-électrodes (A) selon une des revendications précédentes, comprenant une couche de support mécanique (B) pour le substrat (SUB), située à un endroit à l'opposé de la surface apparente des zones d'électrodes (E) et des zones de connexion (P) des pistes conductrices, dans laquelle la couche de base (B) est en verre.

7. Méthode de fabrication d'une grille de micro-électrodes (A), comprenant un substrat isolant (SUB) et un ensemble de pistes conductrices (E, T, P) définies sur ce substrat, comprenant chacune :
- une zone d'électrode (E) de dimensions micrométriques, apparaissant en surface de la grille (A) dans une première zone de contact avec un échantillon biologique à analyser ou à stimuler,
- une zone de connexion (P) de dimensions macroscopiques, apparaissant en surface de la grille (A) dans une deuxième zone de contact pour le branchement électrique avec des circuits externes pour le traitement des signaux, adaptée pour produire des signaux électriques de stimulation à transporter jusqu'à ladite zone d'électrode (E) ou pour traiter des signaux électriques collectés par ladite zone d'électrode (E) représentatifs de l'activité physiologique de l'échantillon et
- une zone d'interconnexion incrustée (T) entre ladite zone d'électrode (E) et ladite zone de connexion (P), la méthode comprenant les étapes de :
- configuration d'un volume de substrat isolant (SUB) d'un premier polymère à l'aide d'un moule principal (M) préétabli portant une configuration de microstructures (S) adaptées pour définir un ensemble de zones d'électrodes (E), des zones de connexion correspondantes (P) et des zones d'interconnexion associées (T) dans le volume du substrat (SUB), sous la forme de cavités (C),
- le remplissage desdites cavités (C) avec un deuxième polymère conducteur de manière à former lesdites pistes conductrices (E, T, P) et
- la disposition d'une couche étanche de matériau isolant (B) pour lesdites cavités remplies (C).

8. Méthode selon la revendication 7, dans laquelle la configuration d'un volume de substrat isolant (SUB) est réalisée par moulage de reproduction et comprend les étapes suivantes :
- dépôt du premier matériau sous une forme visqueuse pré-polymère dans le moule (M),
- réticulation du volume de matériau,
- séparation du volume de substrat (SUB) du moule (M), de manière à rendre accessibles les cavités (C) formées par la configuration de microstructures en relief (S) du moule (M) et
- remplissage des cavités (C) avec le deuxième matériau sous forme liquide pré-polymère par diffusion par capillarité, suivi du séchage du matériau.

9. Méthode selon la revendication 7, dans laquelle le polymère isolant est du polydiméthylsiloxane (PDMS).

10. Méthode selon la revendication 9, comprenant une étape d'hydrophilisation du PDMS à l'aide d'un plasma d'oxygène.

11. Méthode selon la revendication 7, dans laquelle le polymère conducteur est du poly(3,4-éthylènedioxythiophène) (PEDOT).

12. Méthode selon la revendication 7, dans laquelle le moule principal (M) est obtenu par structuration photolithographique d'une première couche de résine photosensible (R) pour la configuration de zones conductrices incrustées (T) de la grille (A) et, ensuite, d'une deuxième couche de résine photosensible superposée (R) pour la configuration des zones conductrices apparentes (AND, P) de la grille (A).

13. Méthode selon la revendication 12, dans laquelle la fabrication du moule principal (M) comprend :
- la préparation d'un substrat de support (W),
- le dépôt d'une première couche de résine photosensible (R) sur le substrat (W),
- l'éclairage de la couche de résine déposée (R) à travers un premier masque photographique (MI) portant l'image desdites zones d'électrodes (E), des zones de connexion correspondantes (P) et des zones d'interconnexion associées (T) pour atteindre un état réticulé (R'),
- le dépôt d'une deuxième couche de résine photosensible (R) sur la première couche,
- l'éclairage de la couche de résine déposée (R) à travers un deuxième masque photographique (M2) dans un état aligné et portant l'image desdites zones d'électrodes (E) et des zones de connexion correspondantes (P) pour atteindre un état réticulé (R') et
- le développement et l'élimination sélective des couches de résine non réticulées (R) de manière à ce que ladite configuration de microstructures (S) soit en relief sur le substrat de base (W).
